(19) Europäisches Patentamt
European Patent Office
Office européen des brevets

(11) Veröffentlichungsnummer: **0 113 432 B1**

(12) **EUROPÄISCHE PATENTSCHRIFT**

(45) Veröffentlichungstag der Patentschrift: **17.03.93**

(21) Anmeldenummer: **83112147.0**

(22) Anmeldetag: **02.12.83**

Die Akte enthält technische Angaben, die nach dem Eingang der Anmeldung eingereicht wurden und die nicht in dieser Patentschrift enthalten sind.

(51) Int. Cl.5: **C07D 215/18**, C07D 215/20, C07D 215/26, C07D 215/25, A61K 31/47

# (54) Neue Chlormethylchinolin-Derivate, Verfahren zu ihrer Herstellung und ihre Verwendung.

(30) Priorität: **02.12.82 HU 386982**

(43) Veröffentlichungstag der Anmeldung: **18.07.84 Patentblatt 84/29**

(45) Bekanntmachung des Hinweises auf die Patenterteilung: **17.03.93 Patentblatt 93/11**

(84) Benannte Vertragsstaaten: **BE CH DE FR GB LI LU NL**

(56) Entgegenhaltungen:
**EP-A- 111 329**
**EP-A- 0 049 776**
**DE-A- 2 458 146**
**FR-A- 2 166 196**

**JOURNAL OF HETEROCYCLIC CHEMISTRY, Band 2, Nr. 2, Juni 1965 ADRIENNE STEINAKKER DEY et al.: "Synthesis and Properties of Fluorine-Containing Heterocyclic Compounds. I. Trifluoromethyl Ouinolines (I)", Seiten 113-119**

(73) Patentinhaber: **ALKALOIDA VEGYéSZETI GYáR**
**Postfach 1**
**H-4440 Tiszavasvári(HU)**

(72) Erfinder: **Salamon, Zoltán, Dipl.-Chem.**
**Elmunkas u.11.**
**Tiszavasvári(HU)**
Erfinder: **Imre geb. Virág, Ilona**
**Kabay J. u.25**
**Tiszavasvári(HU)**
Erfinder: **Sebestyén, Magdolna**
**Irányi u. 16.E.**
**Hajdunánás(HU)**

(74) Vertreter: **Patentanwälte Beetz sen. - Beetz jun. Timpe - Siegfried - Schmitt-Fumian**
**Steinsdorfstrasse 10**
**W-8000 München 22 (DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Rank Xerox (UK) Business Services
(3.10/3.5x/3.0.1)

JOURNAL OF MEDICINAL CHEMISTRY, Band 11, Nr. 2, 26. Februar 1968, The American Chemical Society W.P. PURCELL et al.: "Application of regression analyses to antitumor activitiesof various acetylenic carbamates", Seiten 267-269.

CHEMICAL ABSTRACTS, Band 96, Nr. 9, 1. März 1982, Zusammenfassung Nr. 68776v, Seite 591, Columbus Ohio (US)

JOURNAL OF MEDICINAL CHEMISTRY, Band 14, Nr. 12, Dezember 1971, J.C.CRAIG et al.: "Potential Antimalarials".7. Tribromomethylquinolines and positive halogen compounds", Seiten 1221-1222

THE CHEMISTRY OF HETEROCYCLIC COMPOUNDS: OUINOLINES, Band 32, Teil I, John Wiley & Sons, Londen (GB)

Methoden der Organischen Chemie (Houben-Weyl), Band V/3 Halogenverbindungen, Georg Thieme Verlag, Stuttgart 1962 (4. Auflage)

## Beschreibung

Die Erfindung betrifft neue Halogenmethylchinolin-Derivate der allgemeinen Formel I

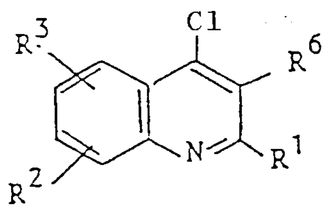

(I),

worin bedeuten:

$R^1$ $-CCl_3$, $-CF_3$, $-CHCl_2$ oder $-CH_2Cl$,

$R^2$ Wasserstoff, Halogen, Methoxy, Methyl, $-CCl_3$, $-CF_3$ oder $CHCl_2$,

$R^3$ Wasserstoff, Halogen, Methoxy, Methyl oder $-CF_3$,

wobei $R^2$ und $R^3$ nicht gleichzeitig Wasserstoff bedeuten können und $R^2$ $-CCl_3$ oder $-CHCl_2$ bedeutet, wenn $R^1$ $-CF_3$ ist, und $R^3$ Wasserstoff oder $-CH_3$ bedeutet, wenn $R^2$ Chlor ist, und $R^2$ Wasserstoff oder $-CH_3$ bedeutet, wenn $R^3$ Chlor ist, sowie 2-Trifluormethyl-4,5-dichlor-8-trichlormethylchinolin.

Die Verbindungen werden erfindungsgemäß hergestellt durch Chlorierung von

a) Chinolinderivaten der allgemeinen Formel II

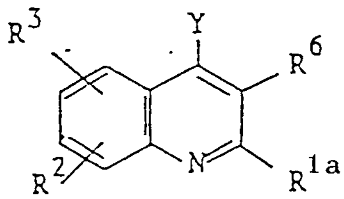

(II),

worin bedeuten:

$R^{1a}$ $-CH_3$, $-CHCl_2$ oder $-CH_2Cl$,

$R^2$ und $R^3$ das gleiche wie oben und

Y Halogen oder Hydroxy,

b) Chinolinderivaten der allgemeinen Formel IV

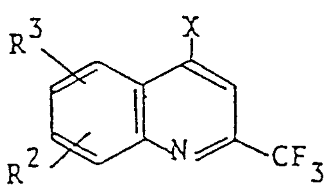

(IV),

worin bedeuten:

$R^2$ und $R^3$ dasselbe wie oben und

X Halogen, vorzugsweise Chlor oder Brom,

oder

c) Chinolinderivaten der allgemeinen Formel V

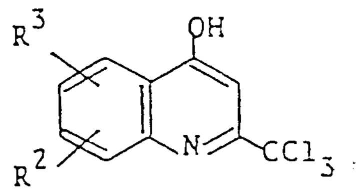

(V)

mit $R^2$ und $R^3$ wie oben, wobei die Ausgangssubstanzen mindestens ein Chloratom weniger aufweisen als die entsprechenden Endprodukte der Formel I.

Ein Teil der erfindungsgemäßen Verbindungen sind pharmazeutische Wirkstoffe, die anderen sind Zwischenprodukte bei der Herstellung von Arzneimittelwirkstoffen.

Aus der Literatur sind ähnliche Verbindungen bekannt, denen eine biologische Wirkung zugeschrieben wird, zum Beispiel eine Wirkung gegen Malaria (J. Med. Chem. 14 (1971) 1221) oder gegen Bakterien (GB-PS 874 980). Es bestand daher Interesse an einem geeigneten Verfahren zur Herstellung von Chlormethylderivaten.

Das bisher zu diesem Zweck allgemein angewandte Verfahren (J. Chem. Soc. 123 (1923) 2882) ist in erster Linie zur Herstellung von 2-Tribrommethylchinolin-Derivaten geeignet (J. Chem. Soc. 1950, 628; loc. cit. 1951, 1145; loc. cit. 1953 (1369) und ergibt bei Anwendung auf 4-Chlorchinaldin-Derivate nur geringe Ausbeuten (J. Med. Chem. 14 (1971) 1221).

Eine vorteilhaftere Methode zur Herstellung von 2-Trichlormethyl-4-chlorchinolin besteht darin, daß man 4-Chinaldinol in Gegenwart von Phosphorpentachlorid in Phosphoroxychlorid kocht. Das Produkt kann aus dem erhaltenen Gemisch in mäßiger Ausbeute chromatographisch abgetrennt werden (Chem. Ph. Bull. 29 - (1981) 1069). Durch Kondensieren von Ethyltrichloracetylacetat mit dem entsprechenden Anilin wurden in mittlerer Ausbeute 2-Trichlormethyl-4-chinolinol-Derivate erhalten (J. Org. Chem. 31 (1966) 3369, für deren Umsetzung zu 4-Chlorderivaten aus der Literatur keine Methode bekannt ist.

Der Nachteil der bisher bekannten Verfahren besteht darin, daß die Ausbeuten gering bis mittelmäßig sind und-sich die Verfahren nur zur. Herstellung der 2-Chlormethylchinolinderivate eignen; in vielen Fällen ist ferner die Selektivität der Reaktionen nicht ausreichend. In manchen Fällen sind auch die Ausgangsverbindungen (zum Beispiel die Trichloracetylessigester) schwer zugänglich und teuer.

Aus EP-A-49 776 sind substituierte 4-Methyl-2-trifluormethylchinolinderivate bekannt, die als Antimalariamittel verwendbar sind.

DE-A-2 458 146 sind 2-Trifluormethylchinolinderivate zu entnehmen, die in 4-Stellung mit einer Chinuclidinylhydroxymethylgruppe substituiert sind und ebenfalls Antimalariawirkung besitzen.

FR-A-2 166 196 betrifft Derivate des 6,7-Dihydro-1-oxo-1H,5H-benzo[ij]-chinolizins, die antimikrobielle Wirksamkeit aufweisen.

In J. Het. Chem. 2 (1965) 113-119 sind substituierte 2-Trifluormethyl-4-chlorchinoline beschrieben, die ggfs. in 6-Stellung mit Br, $CH_3$ oder $-OCH_3$ und in 8-Stellung mit $CH_3$ substituiert sind. Diese Verbindungen erwiesen sich nicht als antimikrobiell wirksam.

In J. Med. Chem. 11 (1968) 267-269 sind 2-Piperidyl-4-hydroxymethylchinoline, die ggfs. 2-trifluormethylsubstituiert sind eine geringe Antimalariawirkung aufweisen und photosensibilisierend wirken, sowie 2-Trifluormethyl-4-halogenchinolinderivate beschrieben, die im homoaromatischen Ring halogen- oder methylsubstituiert sind.

In der Monographie 'Quinolines', Part I, The Chemistry of Heterocyclic Compounds, G. Jones, John Wiley & Sons, 31 (1), 735-738, sind 4-Halogen(halogenalkyl)chinoline aufgelistet; pharmakologische Wirksamkeiten sind zu diesen Verbindungen nicht angegeben.

EF-A— 111 329 sind schließlich 2-fluormethyl- und 2-chlormethylsubstituierte 4-Halogen-chinolinderivate zu entnehmen, die im homoaromatischen Ring ggfs. halogen- oder alkylsubstituiert sind.

Die Erfindung beruht auf der überraschenden Feststellung, daß die Alkylgruppe bzw. die Alkylgruppen von Methyl-4-chlorchinolinen bzw. Methyl-4-oxychinolinen selektiv chlorierbar sind. Als geeignetes Chlorierungsmittel erwies sich Phosphorpentachlorid, das auch aus Phosphortrichlorid und Chlor in situ hergestellt werden kann.

Ein überraschender Vorteil besteht ferner darin, daß geringe Mengen von Phosphorpentahalogeniden oder Phosphortrihalogeniden in Gegenwart eines Lösungsmittels die mit elementarem Chlor vorgenommene Chlorierung der Seitenkette katalysieren, die Reaktion beschleunigen und die Selektivität der Reaktion erhöhen. Auf diese Weise können die homoaromatischen Alkylgruppen des Chinolins in guter Ausbeute di-

bzw trihalogeniert werden.

Gemäß einer bevorzugten Ausführungsform der Verfahrensvariante a) werden daher als Chlorierungsmittel Phosphorpentachlorid oder Phosphoroxychlorid und Phosphorpentachlorid oder Chlorgas oder Phosphortrichlorid und Chlorgas verwendet.

Die Reaktionsbedingungen werden in Abhängigkeit von der Zielverbindung gewählt. Die eine Untergruppe der Verbindungen der allgemeinen Formel I bildenden Verbindungen der allgemeinen Formel VI

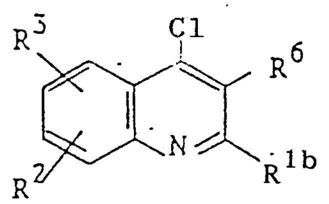

(VI),

worin $R^2$ und $R^3$ dasselbe wie oben und
$R^{1b}$ $-CH_2Cl$, $-CHCl_2$ oder $-CCl_3$ bedeuten,
können zum Beispiel hergestellt werden, indem man Chinoline der allgemeinen Formel II in Halogenkohlenwasserstoffen bei 70 bis 140 °C mit Phosphoroxychlorid chloriert.

Zur Herstellung der eine Untergruppe der Verbindungen der allgemeinen Formel I bildenden Verbindungen der allgemeinen Formel VII

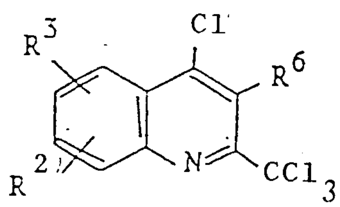

(VII)

mit $R^2$ und $R^3$ wie oben,
werden vorzugsweise Verbindungen der allgemeinen Formel III bei zweckmäßig 150 bis 220 °C mit Phosphortrihalogenid und Chlorgas chloriert, wobei als Lösungsmittel Halogenkohlenwasserstoffe verwendet werden und gegebenenfalls unter Druck gearbeitet wird.

In den beiden genannten Verfahrensvarianten findet als Lösungsmittel Mono-, Di- oder Trichlorbenzol oder Tetrachlorkohlenstoff Verwendung

Zur Herstellung der eine Untergruppe der Verbindungen der allgemeinen Formel I bildenden Verbindungen der allgemeinen Formel VIII

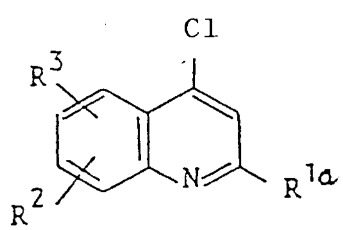

(VIII)

mit $R^{1a}$, $R^2$ und $R^3$ wie oben
wird eine Verbindung der allgemeinen Formel IX

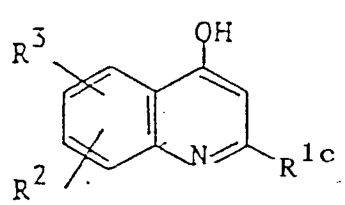

(IX) /

worin $R^2$ und $R^3$ dasselbe wie oben und $R^{1c}$ $-CH_3$, $-CHCl_2$, $-CH_2Cl$, $-CF_3$ oder $-CCl_3$ bedeuten, bei 120 bis 150 °C mit Phosphorpentachlorid chloriert. Vorzugsweise wird als Lösungsmittel ein Halogenkohlenwasserstoff, zweckmäßig ein Chlorbenzol verwendet. In der obigen Formel IX sowie im folgenden bedeutet $R^{16}$ $-CH_3$, $-CHCl_2$, $-CH_2Cl$, $-CF_3$ oder $-CCl_3$.

Die eine Untergruppe der Verbindungen der allgemeinen Formel I bildenden Verbindungen der allgemeinen Formel XI

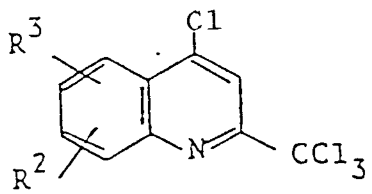

(XI)

mit $R^2$ und $R^3$ wie oben

werden zweckmäßig gemäß der Variante b) des erfindungsgemäßen Verfahrens hergestellt. Zu diesem Zweck wird ein Chinolinderivat der allgemeinen Formel IV mit Aluminiumchlorid umgesetzt, wobei man als Lösungsmittel zweckmäßig Schwefelkohlenstoff, Nitrobenzol oder Acetylchlorid verwendet.

Die Verfahrensvariante c) ist zur Herstellung der eine Untergruppe der Verbindungen der allgemeinen Formel I bildenden Verbindungen der allgemeinen Formel XI

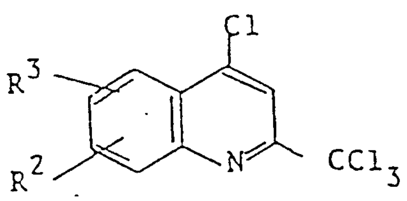

(XI)

mit $R^2$ und $R^3$ wie oben

geeignet. Dazu wird eine Verbindung der allgemeinen Formel V mit Phosphoroxychlorid oder bei 20 bis 100 °C mit Phosphorpentachlorid umgesetzt.

Die Halogenmethylchinolinderivate der allgemeinen Formel I sind als Zwischenprodukte bzw. Wirkstoffe zur Herstellung von Arznei- und Pflanzenschutzmitteln geeignet.

Die erfindungsgemäßen pharmazeutischen Mittel, die sich zur human- und veterinärmedizinischen Anwendung eignen, enthalten mindestens eine Verbindung der Formel

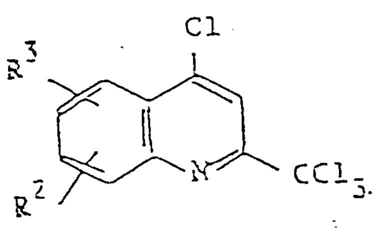

(XI)

worin die Substituenten $R^2$ und $R^3$ in den nachstehend angegebenen Kombinationen folgende Bedeutung besitzen:

| $R^2$ | $R^3$ |
|---|---|
| 8-MeO | H |
| 8-Me | H |
| 8-$CCl_3$ | H |
| 5-Cl | 8-Me |
| 6-$CF_3$ | H |
| 6-MeO | H |
| 8-$CF_3$ | H |
| 6-Me | H |
| 8-$CHCl_2$ | H , |

als Wirkstoff, zusammen mit üblichen Hilfs- und/oder Trägerstoffen. Sie sind insbesondere als Fungicide, Antiphlogistica und Analgetica verwendbar.

Das erfindungsgemäße Verfahren wird im folgenden anhand von Beispielen näher erläutert.

Die für die Umsetzungen benötigten Chinolinderivate wurden nach aus der Literatur bekannten, allgemein üblichen Verfahren hergestellt, so zum Beispiel die 2-Methyl-4-hydroxychinolinderivate nach der von Hauser und Reynold (J. Am. Chem. Soc. 70 (1948) 2402 modifizierten Conrad-Limpach-Synthese, das entsprechende 2-Trifluormethyl-4-chinolin nach der von A.S.Dey und M.M. Joullie (J. Het. Chem. 2 (1965) 113) angegebenen Methode. Aus diesem wurden die entsprechenden 2-Trifluormethyl-4-halogen-Derivate durch Erwärmen in Phosphoroxyhalogeniden gewonnen.

Beispiel 1

2-Trichlormethyl-4,8-dichlorchinolin

Ein Gemisch von 1,9 g 2-Methyl-8-chlor-4-hydroxychinolin und 9,0 g Phosphorpentachlorid in 20 ml Chlorbenzol wird auf 130 bis 135 °C erwärmt und bei dieser Temperatur 2 Stunden lang gerührt. Das Reaktionsgemisch wird im Vakuum eingedampft und der Rückstand aus 20 ml Methanol umkristallisiert. Es werden 2,01 g (64,5 %) Produkt erhalten. F.: 126-128 °C.

Beispiel 2

2-Trichlormethyl-6-methyl-4-chlorchinolin

Ein Gemisch von 1,9 g 2,6-Dimethyl-4-chlorchinolin und 6,5 g Phosphorpentachlorid in 20 ml Phosphoroxychlorid wird unter Rühren 3 Stunden lang gekocht. Dann wird das Gemisch in Eiswasser gegossen. Das ausgeschiedene Produkt wird abfiltriert. Ausbeute: 2,81 g (95,1 %). F.: 85-88 °C.

Beispiel 3

2-Trichlormethyl-8-trifluormethyl-4-chlorchinolin

14,5 g 2-Methyl-8-trifluormethyl-4-hydroxychinolin werden unter ständigem Rühren in 70 ml Phosphoroxychlorid 20 Minuten lang gekocht. Das Gemisch wird dann mit Wasser auf 70 °C abgekühlt und mit 10 ml Phosphortrichlorid versetzt; anschließend wird bei 95 bis 100 °C 90 Minuten lang Chlorgas durch das Gemisch geleitet. Schließlich wird das Reaktionsgemisch unter vermindertem Druck eingedampft, der Rückstand mit Eiswasser behandelt und das ausgeschiedene Produkt abfiltriert. Ausbeute: 22,1 g. (99,1 %) F.: 88-90 °C.

Beispiel 4

2-Chlormethyl-8-methyl-4-chlorchinolin und 2-Dichlormethyl-8-methyl-4-chlorchinolin

Ein Gemisch von 1,9 g 2,8-Dimethyl-4-chlorchinolin und 2,1 g Phosphorpentachlorid in 5 ml Tetrachlorkohlenstoff wird unter ständigem Rühren 2,5 Stunden lang gekocht. Die nach Abkühlen auf Raumtemperatur und Stehenlassen über Nacht ausgeschiedene Substanz wird abfiltriert. Sie besteht aus einem Gemisch aus dem Ausgangsstoff (30 mol-%) und dem Hydrochlorid des 2-Chlormethyl-8-methyl-4-chlorchinolins (25 mol-

%). Das Filtrat wird im Vakuum eingedampft und der Rückstand säulenchromatographisch (Silikagel, Elutionsmittel Cyclohexan) aufgearbeitet.

In der ersten Fraktion findet sich 2-Trichlormethyl-8-methyl-4-chlorchinolin (10 mol-%); danach wird 2-Dichlormethyl-8-methyl-4-chlorchinolin eluiert (23 mol-%). Als letzte Fraktion erholt man 2-Chlormethyl-8-methyl-4-chlorchinolin (10 mol-%).

Beispiel 5

2-Trichlormethyl-8-trifluormethyl-4-chlorchinolin

Ein Gemisch aus 3,4 g 2,8-Bis(trifluormethyl)-4-bromchinolin, 1,5 g frisch sublimiertem Aluminiumchlorid und 30 ml wasserfreiem Schwefelkohlenstoff wird bei Raumtemperatur über Nacht stehengelassen. Dann wird die Lösung mit 50 ml eiskalter 10 %iger Salzsäure und anschließend mit 2 x 50 ml Wasser gewaschen, getrocknet und dann eingedampft. Der Rückstand wird aus wasserfreiem Methanol umkristallisiert. Ausbeute: 3,3 g (86,9 %) F.: 88-90 °C

Beispiel 6 .

2-Trichlormethyl-8-dichlormethyl-4-chlorchinolin

Ein Gemisch von 1,92 g 2,8 Dimethyl-4-chlorchinolin, 15,0 g Phosphorpentachlorid und 100 ml c-Dichlorbenzol wird 4 Stunden lang auf 175 °C gehalten. Das bei der Reaktion entstehende Phosphortrichlorid wird laufend abdestilliert. Das Reaktionsgemisch wird dann auf die im Beispiel 1 beschriebene Weise aufgearbeitet. Ausbeute: 3,1 g (85,2 %) F.: 95-97 °C.

Beispiel 7

2,6-Bis(trichlormethyl)-4-chlorchinolin

Ein Gemisch aus 8,66 g 2,6-Dimethyl-4-hydroxychinolin und 30 ml Phosphoroxychlorid wird am Rückfluß 30 Minuten lang gekocht, dann auf 70 °C abgekühlt und mit Hexan versetzt. Das kristallin ausfallende 2,6-Dimethyl-4-chlorchinolin-hydrochlorid wird abfiltriert und mit wenig eiskaltem Aceton und dann mit Tetrachlorkohlenstoff angeteigt. Die Substanz wird zusammen mit 75 g Phosphorpentachlorid 18 Stunden lang auf 180 °C gehalten. Die erhaltene Schmelze wird in der üblichen Weise aufgearbeitet. Ausbeute: 9,7 g (48,7 %). F.: 92-94 °C.

Beispiel 8

2,6-Bis(trichlormethyl)-4-chlorchinolin

Eine Lösung von 29,5 g 2-Trichlormethyl-6-methyl-4-chlorchinolin in 100 ml 1,2,4-Trichlorbenzol wird mit 0,5 ml Phosphortribromid versetzt und unter Rühren auf 100 °C erhitzt.Dann wird ein intensiver Chlorstrom durch das Reaktionsgemisch geleitet. Danach wird weiter auf 200 °C erwärmt und bei dieser Temperatur weitere 2 Stunden lang chloriert. Das Reaktionsgemisch wird anschließend gemäß Beispiel 8 aufgearbeitet. Ausbeute: 36,3 g (91,2 %). F.: 92-94 °C.

Beispiel 9

2,8-Bis(trichlormethyl)-4-chlorchinolin

In einem doppelwandigen, mit Rührer ausgerüsteten Hastelloy-C-Autoklaven von 1 Liter Inhalt werden 233 ml Tetrachlorkohlenstoff und 80 ml Phosphortrichlorid vorgelegt. Dann werden bei 80 °C 55 g Chlorgas in das Gemisch geleitet, worauf noch eine halbe Stunde lang gerührt und dann mit 17,3 g 2,8-Dimethyl-4-hydroxychinolin versetzt wird. Die Temperatur des Reaktionsgemisches wird langsam auf 180 °C erhöht und das Gemisch bei dieser Temperatur 24 Stunden lang gerührt. Dabei steigt der Innendruck auf 28 bis 29 bar an Das Lösungsmittel wird abdestilliert und der Rückstand auf die in Beispiel 1 beschriebene Weise aufgearbeitet. Ausbeute: 33,2 g (83,4 %).

Beispiel 10

2,8-Bis(trichlormethyl)-4-chlorchinolin

Ein Gemisch von 47,9 g 2,8-Dimethyl-4-chlorchinolin und 200 ml 1,2,4-Trichlorbenzol wird auf 95 °C erwärmt und mit 1 ml Phosphortribromid versetzt; dann wird unter intensivem Rühren Chlor durch das Gemisch geleitet. Man läßt danach die Temperatur auf 130 °C ansteigen und rührt bei dieser Temperatur 15 Minuten, dann weitere 4 Stunden bei 200 °C. Das Lösungsmittel wird im Vakuum abdestilliert und der Rückstand aus 400 ml Ethanol umkristallisiert. Ausbeute: 85,6 g (85,8 %). F.: 134-136 °C.

Beispiel 11

2-Trichlormethyl-4,6-dichlorchinolin

Ein Gemisch aus 3,0 g 2-Trichlormethyl-6-chlor-4-hydroxychinolin und 20 ml Phosphoroxychlorid wird 3 Stunden lang am Rückfluß gekocht. Das Gemisch wird unter verminderten Druck eingedampft und der Rückstand in üblicher Weise aufgearbeitet. Ausbeute: 2,2 g (69,8 %). F.: 99-101 °C.

Beispiele 12 bis 39

In der nachstehenden Tabelle 1 sind weitere erfindungsgemäße Verbindungen aufgelistet, die nach dem jeweils angegebenen Verfahren hergestellt wurden.

Tabelle 1

| Beispiel | Verbindung | Verfahren gemäß Beispiel | Ausbeute (%) |
|---|---|---|---|
| 12 | 2-Trichlormethyl-5-methyl-4-chlorchinolin | 1 | 74,7 |
| 13 | 2-Trichlormethyl-6-methyl-4-chlorchinolin | 1 | 57,3 |
| 14 | 2-Trichlormethyl-8-methyl-4-chlorchinolin | 2 | 87,3 |
| 15 | 2-Trichlormethyl-4,5-dichlor-8-methylchinolin | 1 | 81,8 |
| 16 | 2-Trichlormethyl-4,6-dichlorchinolin | 1 | 83,9 |
| 17 | 2-Trichlormethyl-4,7-dichlorchinolin | 1 | 63,4 |
| 18 | 2-Trichlormethyl-6-methoxy-4-chlorchinolin | 1 | 64,3 |

Tabelle 1, Fortsetzung

| Beispiel | Verbindung | Verfahren gemäß Beispiel | Ausbeute (%) |
|---|---|---|---|
| 19 | 2-Trichlormethyl-6-methoxy-4-chlorchinolin | 2 | 69,6 |
| 20 | 2-Trichlormethyl-8-methoxy-4-chlorchinolin | 1 | 48,2 |
| 21 | 2-Trichlormethyl-6-trifluormethyl-4-chlorchinolin | 2 | 94,3 |
| 22 | 2-Trichlormethyl-6-trifluormethyl-4-chlorchinolin | 5 | 73,7 |
| 23 | 2-Trichlormethyl-7-trifluormethyl-4-chlorchinolin | | 79,8 |
| 24 | 2-Trichlormethyl-7-trifluormethyl-4-chlorchinolin | 5 | 84,5 |
| 25 | 2-Trichlormethyl-8-trifluormethyl-4-chlorchinolin | | 87,5 |
| 26 | 2-Trichlormethyl-8-trifluormethyl-4-chlorchinolin | | 71,4 |
| 27 | 2-Trichlormethyl-8-trifluormethyl-4-chlorchinolin | 5 | 78,3 |
| 28 | 2,6-Bis(trichlormethyl)-4-chlorchinolin | 10 | 87,6 |
| 29 | 2-Trifluormethyl-4-chlor-5-dichlormethylchinolin | 8 | 73,2 |
| 30 | 2-Trifluormethyl-4-chlor-6-trichlormethylchinolin | 8 | 87,4 |
| 31 | 2-Trifluormethyl-4-chlor-7-trichlormethylchinolin | 8 | 81,7 |
| 32 | 2-Trifluormethyl-4-chlor-8-dichlormethylchinolin | 8 | 79,3 |
| 33 | 2-Trifluormethyl-4,5-dichlor-8-trichlormethylchinolin | 8 | 84,6 |
| 34 | 2-Trifluormethyl-4-chlor-8-trichlormethylchinolin | 8 | 67,3 |

Tabelle 1, Fortsetzung

| Beispiel | Verbindung | Verfahren gemäß Beispiel | Ausbeute % |
|---|---|---|---|
| 35 | 2-Trichlormethyl-4-chlor-7-methylchinolin | 1 | 73,2 |
| 36 | 2-Trichlormethyl-4,6-dichlor-8-methylchinolin | 1 | 86,3 |
| 37 | 2-Trichlormethyl-4,7-dichlor-8-methylchinolin | 1 | 93,4 |
| 38 | 2-Trichlormethyl-4,8-dichlor-6-methylchinolin | 1 | 73,7 |
| 39 | 2-Dichlormethyl-4,8-dichlor-6-methylchinolin | | 65,3 |

In der folgenden Tabelle 2 sind physikalische Konstanten der in den Beispielen hergestellten Verbindungen aufgeführt.

Die Schmelzpunktwerte sind nicht korrigiert. Die NMR-Spektren wurden in $CDCl_3$ aufgenommen; bei den angegebenen Werten ist die erste Stelle nach dem Komma geschätzt. Die Verschiebungen sind auf Tetramethylsilan als Standard bezogen.

Verwendete Abkürzungen:

- s Singulett
- d Doublett
- t Triplett
- q Quartett
- m Multiplett
- b breites Signal
- $J^x$ Kopplungskonstante, wobei der obere Index (zB x) die Anzahl der Bindungen angibt, über die die Kopplung wirkt. Bei heteronuklearer Kopplung weist der Index rechts unten auf die Kopplung, zum Beispiel F-H, hin.

Tabelle 2

| Beispiel | $R^1$ | $R^3$ | $R^2$ | F. (°C) | $^1$H-NMR-Daten (ppm) |
|---|---|---|---|---|---|
| 12 | $CCl_3$ | 5-Me | H | 89-92 | 8,10 s (1H) H (3); 3,02 s (3H) 5-Methyl, 8,07 dd (1H); 7,70 dd (1H); 7,47 dd (1H); 6-, 7- und 8-H. |
| 35 | $CCl_3$ | 7-Me | H | 82-83 | 8,13 d ($J^3$ 8,5 Hz 1H) H (5); 8,09 s (1H) H (3); 8,00 s(b) (1H) H (8); 7,57 dd ($J^3$ 8,5 Hz, $J^4$ 1,5 Hz 1H) H (6); 2,61 s (3H) 7-Methyl. |
| 14 | $CCl_3$ | 8-Me | H | 104-106 | 8,14 s (1H) H (3); 8,08 dd ($J^3$ 8,5 Hz; $J^4$ 1,5 Hz, 1H) H (5); 7,68 dm (1H) H (7); 7,60 dd ($J^3$ 8,5 Hz; $J^{3'}$ 2,5 Hz; 1H) H (6); 2,87 s (3H) 8-Methyl. |
| 4 | $CHCl_2$ | 8-Me | H | 104-107 | 8,10 dd ($J^3$ 8,0 Hz; $J^4$ 0,9 Hz, 1H) H (5); 7,97 s (1H) H (3); 7,60 dd ($J^3$ 8,0 Hz; $J^{3'}$ 7,6 Hz, 1H) H (6); 7,66 dm ($J^3$ 7,6 Hz, 1H) H (7); 6,85 s (1H) 2-Dichlormethyl H; 2,75 s (3H) 8-Methyl. |
| 4 | $CH_2Cl$ | 8-Me | H | 69-72 | 8,08 dd (1H) H (5); 7,70 s (1H) H (3); 7,62 d (1H) H (7); 7,52 dd (1H) H (6); 4,85 s (2H) Chlormethyl-H; 2,80 s (3H) 8-Methyl. |

Tabelle 2, Fortsetzung

| Beispiel | $R^1$ | $R^3$ | $R^2$ | F. (°C) | $^1$H-NMR-Daten (ppm) |
|---|---|---|---|---|---|
| 21, 22 | $CCl_3$ | 6-$CF_3$ | H | 47-48 | 8,56 dq ($J^4$ 2,0 Hz; $J^4_{F-H}$ 0,9 Hz, 1H) H (5); 8,35 dq ($J^3$ 9,0 Hz; $J^5_{F-H}$ 0,6 Hz, 1H) H (8); 8,25 s (1H) H (3); 8,03 dd(b) ($J^3$ 9,0 Hz; $J^4$ 7,0 Hz, 1H) H (7). |
| 23, 24 | $CCl_3$ | 7-$CF_3$ | H | 72-74 | 8,55 dq ($J^4$ 1,8 Hz; $J^4_{F-H}$ 0,9 Hz; 1H) H (8); 8,39 dq ($J^3$ 9,0 Hz; $J^5_{F-H}$ 0,8 Hz; 1H) H (5); 8,27 s (1H) H (3); 7,96 dd(b) ($J^3$ 9,0 Hz; $J^4$ 1,8 Hz (1H) H (6). |
| 25 | $CCl_3$ | H | 8-$CF_3$ | 88-90 | 8,50 dd ($J^3$ 8,5 Hz; $J^4$ 1,5 Hz, 1H) H (5); 8,28 s (1H) H (3); 8,24 d(b) ($J^3$ 8,5 Hz, 1H) H (7); 7,85 dd ($J^3$ 8,5 Hz; $J^3$: 8,5 Hz 1H) H (6). |
| 11, 16 | $CCl_3$ | H | 6-Cl | 99-101 | 8,24 d ($J^4$ 2,0 Hz; 1H) H (5); 8,18 s (1H) H (3); 8,17 d ($J^3$ 8,5 Hz; 1H) H (8); 7,78 dd ($J^3$ 8,5 Hz $J^4$ 2,0 Hz, 1H) H (7). |

Tabelle 2, Fortsetzung

| Beispiel | $R^1$ | $R^3$ | $R^2$ | F. (°C) | $^1$H-NMR-Daten (ppm) |
|---|---|---|---|---|---|
| 17 | $CCl_3$ | H | 7-Cl | 82-84 | 8,25 dd ($J^4$ 2,0 Hz; $J^5$ 0,4 Hz; 1H) H (8); 8,20 d (b) ($J^3$ 9,0 Hz 1H) H (5); 8,15 s (1H) H (3); 7,69 dd ($J^3$ 9,0 Hz; $J^4$ 2,0 Hz; 1H) H (6). |
| 1 | $CCl_3$ | H | 8-Cl | 126-128 | 8,22 s (1H) H (3); 8,18 dd ($J^3$ 8,7 Hz; $J^4$ 1,5 Hz 1H) H (5); 7,99 dd ($J^3$ 7,8 Hz; $J^4$ 1,5 Hz; 1H) H (7); 7,65 dd ($J^3$ 8,7 Hz; $J^{3'}$ 7,8 Hz 1H) H (6). |
| 20 | $CCl_3$ | H | 8-MeO | 148-151 | 8,18 s (1H) H (3); 7,81 dd ($J^3$ 8,85 Hz; $J^4$ 1,3 Hz, 1H) H (5); 7,65 dd ($J^3$ 8,85 Hz; $J^{3'}$ 7,75 Hz; 1H) H (6); 7,18 dd ($J^3$ 7,75 Hz; $J^4$ 1,3 Hz, 1H) H (7); 4,12 s (3H) 8-Methoxy H. |

Tabelle 2, Fortsetzung

| Beispiel | $R^1$ | $R^3$ | $R^2$ | F. (°C) | $^1$H-NMR-Daten (ppm) |
|---|---|---|---|---|---|
| 15 | $CCl_3$ | 5-Cl | 8-Me | 134-136 | 8,15 s (1H) H (3); 8,65 d (1H) und 7,55 d (1H) H (6 und 7); 280 s (3H) 8-Methyl |
| 36 | $CCl_3$ | 6-Cl | 8-Me | 123-125 | 8,15 s (1H) H (3); 8,10 d ($J^4$ 2,0 Hz, 1H) H (5); 7,65 m (1H) H (7); 2,83 s (3H) 8-Methyl |
| 37 | $CCl_3$ | 7-Cl | 8-Me | 138-139,5 | 8,13 s (1H) H (3); 8,05 d ($J^3$ 9,0 Hz; 1H) und 7,70 d ($J^3$ 9,0 Hz, 1H) H (5 und 7) 2,92 s (3H) 8-Methyl |
| 38 | $CCl_3$ | 6-Me | 8-Cl | | 8,17 s (1H) H (3); 7,93 d ($J^4$ 1,8 Hz; 1H) H (5); 7,80 d (1,8 Hz, 1H) H (7); 2,60 s (3H) 6-Methyl |
| 39 | $CHCl_2$ | 6-Me | 8-Cl | | 8,03 s (1H) H (3); 7,93 d ($J^4$ 1,8 Hz, 1H) H (5); 7,75 d ($J^4$ 1,8 Hz, 1H) H (7); 6,90 s (1H) 2-Dichlormethyl H; 2,58 s (3H) 6-Methyl |
| 7, 8 | $CCl_3$ | H | 6-$CCl_3$ | 92-94 | 8,80-8,83 m (1H) H (5); 8,34-8,32 m (2H) H (7 und 8); 8,25 s (1H) H (3) |
| 6 | $CCl_3$ | H | 8-$CHCl_2$ | 95-97 | 8,45 dd (1H) und 8,35 dd (1H) H (5 und 7); 8,225 s (1H) und 8,22 s (1H) 3- und 8-Dichlormethyl H; 7,85 dd (1H) H (6) |
| 9 | $CCl_3$ | H | 8-$CCl_3$ | 134-136 | 8,58 dd ($J^3$ 7,5 Hz, $J^4$ 1Hz, 1H) und 8,44 dd ($J^3$ 9,0 Hz, $J^4$ 1,0 Hz, 1H) H (5 und 7); 8,24 s (1H) H (3); 7,75 dd ($J^3$ 9,0 Hz, $J^3$ 7,5 Hz 1H) H (6). |

Pharmakologische Wirksamkeit Antimikrobielle Wirksamkeit

Die antimikrobielle Wirksamkeit wurde an folgenden Mikroorganismen untersucht: Bacillus subtilis, E. coli, Proteus vulgaris, Salmonella thyphi murium, Streptococcus faecalis, Staphylococcus aureus, Aspergillus niger, Aspergillus fumigatus, Candida albicans, Saccharomyces cerevisiae, Trichophyton mentagrophytes,

Als Nährböden wurden übliche, mit 2 % Agar verfestigte Nährböden aus Bouillon bzw Sabouraud-Glucose-Pepton verwendet. Die Testverbindungen wurden in Dimethylsulfoxid gelöst; je 0,1 ml der Lösung wurde auf den auf 40 °C abgekühlten Nährboden (pH 7,4) aufgebracht. Die Petrischalen mit den so präparierten Nährböden wurden mit Suspensionen von etwa $10^6$ Zellen/ml infiziert, bei 37 °C im Wärmeschrank inkubiert und am zweiten sowie am siebenten Tag bewertet. Die Kontrollen wurden auf gleiche Weise hergestellt und behandelt, wobei der einzige Unterschied darin bestand, daß sie keinen Wirkstoff enthielten.

Bei den Versuchen ergab sich, daß die Verbindungen der allgemeinen Formel I eine schwache fungicide Wirkung besitzen. Die minimalen Hemmkonzentrationen liegen zwischen 300 und 500 µg/ml.

Entzündungshemmende und analgetische Wirksamkeit

Die entzündungshemmende Wirksamkeit wurde nach der Methode der Hemmung des Carrageenan-Pfotenödems der Ratte (C.A.Winter et al., J. Pharmacol. Exp. Ther. 141 (1963) 369) ermittelt. Zur Bestimmung der analgetischen Wirkung wurde die Methode von Callier (H. Callier et al., Brit. J. Pharmacol. Chemother. 32 (1968) 295) herangezogen, bei der die Hemmung des mit 3-%iger Essigsäure ausgelösten Writhing-Syndroms gemessen wird.

Die erhaltenen Ergebnisse sind in Tabelle 3 zusammengefaßt. Als Referenzwerte sind die Wirksamkeiten von Acetylsalicylsäure und Phenylbutazon im gleichen Test angegeben.

Tabelle 3

| Verbindung der Formel I mit $R^1 = CCl_3$ und $R^6 = H$: $R^2$ | $R^3$ | Dosis i.p. (mg/kg) | Hemmung des Carr.-Ödems (%) | Analgetische Wirksamkeit (%) |
|---|---|---|---|---|
| 8-MeO | H | 100 | 65 | 100 |
| 8-Me | H | 100 | 61 | 60 |
| 8-$CCl_3$ | H | 100 | 50 | 60 |
| 5-Cl | 8-Me | 100 | 47 | 100 |
| 6-$CF_3$ | H | 100 | 33 | 80 |
| 6-MeO | H | 50 | 25 | 60 |
| 8-$CF_3$ | H | 100 | - | 40 |
| 6-Me | H | 100 | - | 40 |
| 8-$CHCl_2$ | H | 100 | - | 40 |
| Acetylsalicylsäure | | 100 | 71 | 60 |
| Phenylbutazon | | 100 | 65 | 20 |

## Patentansprüche

**1.** Halogenmethylchinolinderivate der allgemeinen Formel I

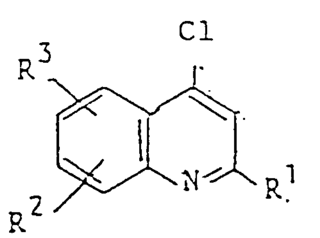

(I),

worin bedeuten:

- $R^1$ $-CCl_3$, $-CF_3$, $-CHCl_2$ oder $-CH_2Cl$,
- $R^2$ Wasserstoff, Halogen, Methoxy, Methyl, $-CCl_3$, $-CF_3$ oder $-CHCl_2$,
- $R^3$ Wasserstoff, Halogen, Methoxy, Methyl oder $-CF_3$,

wobei $R^2$ und $R^3$ nicht gleichzeitig Wasserstoff bedeuten können, $R^2$ $-CCl_3$ oder $-CHCl_2$ bedeutet, wenn $R^1$ $-CF_3$ ist, und $R^3$ Wasserstoff oder $-CH_3$ bedeutet, wenn $R^2$ Chlor ist, und $R^2$ Wasserstoff oder $-CH_3$ bedeutet, wenn $R^3$ Chlor ist, sowie
2-Trifluormethyl-4,5-dichlor-8-trichlormethylchinolin.

**2.** 2-Trichlormethyl-4,8-dichlorchinolin,
2-Trichlormethyl-6-methyl-4-chlorchinolin,
2-Trichlormethyl-8-trifluormethyl-4-chlorchinolin,
2-Chlormethyl-8-methyl-4-chlorchinolin,
2-Dichlormethyl-8-methyl-4-chlorchinolin,
2-Trichlormethyl-8-dichlormethvl-4-chlorchinolin,
2,6-Bis(trichlormethyl)-4-chlorchinolin,
2,8-Bis(trichlormethyl)-4-chlorchinolin,
2-Trichlormethyl-4,6-dichlorchinolin,
2-Trichlormethyl-5-methyl-4-chlorchinolin,
2-Trichlormethyl-8-methyl-4-chlorchinolin,
2-Trichlormethyl-4,5-dichlor-8-methylchinolin,
2-Trichlormethyl-4,7-dichlorchinolin,
2-Trichlormethyl-6-methoxy-4-chlorchinolin,
2-Trichlormethyl-8-methoxy-4-chlorchinolin,
2-Trichlormethyl-6-trifluormethyl-4-chlorchinolin,
2-Trichlormethyl-7-trifluormethyl-4-chlorchinolin,
2-Trifluormethyl-4-chlor-5-dichlormethylchinolin,
2-Trifluormethyl-4-chlor-6-trichlormethylchinolin,
2-Trifluormethyl-4-chlor-7-trichlormethylchinolin,
2-Trifluormethyl-4-chlor-8-dichlormethylchinolin,
2-Trifluormethyl-4,5-dichlor-8-trichlormethylchinolin,
2-Trifluormethyl-4-chlor-8-trichlormethylchinolin,
2-Triflormethyl-4-chlor-7-methylchinolin,
2-Trichlormethyl-4,6-dichlor-8-methylchinolin,
2-Trichlormethyl-4,7-dichlor-8-methylchinolin,
2-Trichlormethyl-4,8-dichlor-6-methylchinolin und
2-Dichlormethyl-4,8-dichlor-6-methylchinolin.

**3.** Halogenmethylchinolinderivate nach Anspruch 1 der Formel (XI)

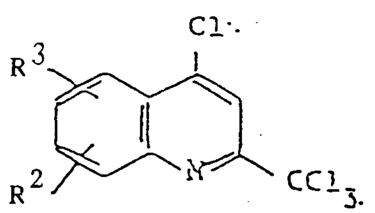

(XI),

worin die Substituenten $R^2$ und $R^3$ in den nachstehend angegebenen Kombinationen folgende Bedeutung besitzen:

| $R^2$ | $R^3$ |
|---|---|
| 8-MeO | H |
| 8-Me | H |
| 8-$CCl_3$ | H |
| 5-Cl | 8-Me |
| 6-$CF_3$ | H |
| 6-MeO | H |
| 8-$CF_3$ | H |
| 6-Me | H |
| 8-$CHCl_2$ | H. |

**4.** Verfahren zur Herstellung der Halogenmethylchinolinderivate der allgemeinen Formel I nach Anspruch 1, gekennzeichnet durch Chlorierung von

a) Chinolinderivaten der allgemeinen Formel II,

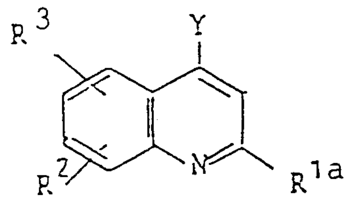

(II),

worin bedeuten:

$R^{1a}$ -$CH_3$, -$CHCl_2$ oder -$CH_2Cl$,

$R^2$ und $R^3$ dasselbe wie in Anspruch 1 und

Y Halogen oder Hydroxy,

b) Chinolinderivaten der allgemeinen Formel IV

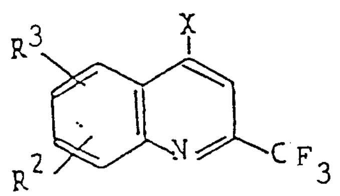

(IV),

worin bedeuten:

$R^2$ und $R^3$ dasselbe wie oben und

X Halogen, vorzugsweise Chlor oder Brom,

oder

c) Chinolinderivaten der allgemeinen Formel V

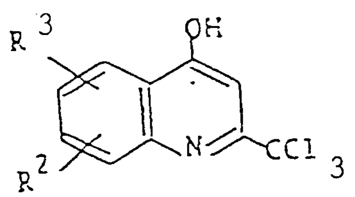

(V),

mit $R^2$ und $R^3$ wie oben,

wobei die Ausgangssubstanzen mindestens ein Chloratom weniger aufweisen als die entsprechenden Endprodukte der Formel I.

5. Verfahren nach Anspruch 4 a), dadurch gekennzeichnet, daß als Chlorierungsmittel Phosphorpentachlorid oder Phosphoroxychlorid und Phosphorpentachlorid oder Chlorgas oder Phosphortrichlorid und Chlorgas eingesetzt werden.

6. Verfahren nach Anspruch 5 zur Herstellung von Chinolinderivaten der allgemeinen Formel VI,

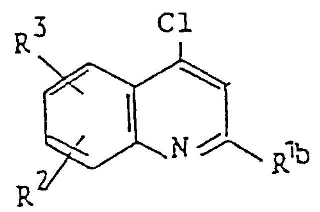

(VI),

worin bedeuten:

$R^2$ und $R^3$ dasselbe wie in Anspruch 4 und

$R^{1b}$ $-CH_2Cl$, $-CHCl_2$ oder $-CCl_3$,

gekennzeichnet durch

Chlorierung eines Chinolinderivats der allgemeinen Formel II nach Anspruch 4 mit Phosphoroxychlorid bei 70 bis 140 °C in Gegenwart von Halogenkohlenwasserstoffen als Lösungsmittel.

7. Verfahren nach Anspruch 5 zur Herstellung von Chinolinderivaten der allgemeinen Formel VII

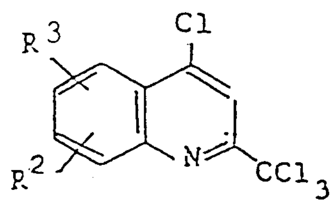

(VII),

mit $R^2$ und $R^3$ wie in Anspruch 4,

gekennzeichnet durch

Chlorierung von Chinolinderivaten der allgemeinen Formel II nach Anspruch 4 bei 150 bis 220 °C und ggf. unter Druck in chlorierten Kohlenwasserstoffen mit einem Phosphortrihalogenid und Chlorgas.

8. Verfahren nach Anspruch 6 oder 7, dadurch gekennzeichnet, daß als Lösungsmittel Chlorbenzol, Dichlorbenzol, Trichlorbenzol oder Tetrachlorkohlenstoff verwendet werden.

9. Verfahren nach Anspruch 5 zur Herstellung von Chinolinderivaten der allgemeinen Formel VIII

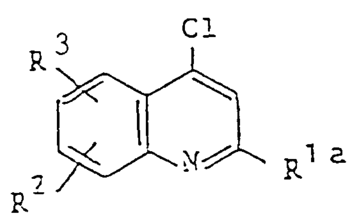

(VIII),

mit $R^{1a}$, $R^2$ und $R^3$ wie in Anspruch 4,

gekennzeichnet durch

Chlorierung eines Chinolinderivats der allgemeinen Formel IX

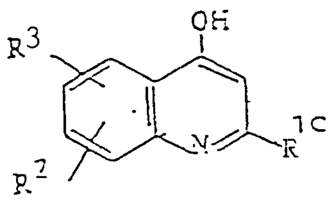

(IX),

worin bedeuten:

$R^2$ und $R^3$ dasselbe wie in Anspruch 4 und

$R^{1c}$ $-CH_3$, $-CHCl_2$, $-CH_2Cl$, $-CF_3$ oder $-CCl_3$,

bei 120 bis 150 °C mit Phosphorpentachlorid.

**10.** Verfahren nach Anspruch 9, gekennzeichnet durch Chlorierung in Gegenwart eines Halogenkohlenwasserstoffes.

**11.** Verfahren nach Anspruch 4 b) zur Herstellung von Chinolinderivaten der allgemeinen Formel XI

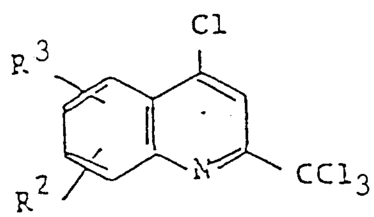

(XI),

mit $R^2$ und $R^3$ wie in Anspruch 1 und insbesondere wie in Anspruch 3,
gekennzeichnet durch
Umsetzung von Chinolinderivaten der allgemeinen Formel IV, worin $R^2$ und $R^3$ dasselbe wie in Anspruch 4 bedeuten, mit Aluminiumchlorid.

**12.** Verfahren nach Anspruch 11, gekennzeichnet durch Umsetzung in Gegenwart von Schwefelkohlenstoff, Nitrobenzol oder Acetylchlorid als Lösungsmittel.

**13.** Verfahren nach Anspruch 4 c) zur Herstellung von Chinolinderivaten der allgemeinen Formel XI

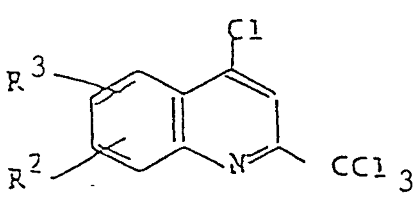

(XI),

mit $R^2$ und $R^3$ wie in Anspruch 1 und insbesondere wie in Anspruch 3,
gekennzeichnet durch Chlorierung von Verbindungen der allgemeinen Formel V, worin $R^2$ und $R^3$ dasselbe wie in Anspruch 3 bedeuten, mit Phosphoroxychlorid oder bei 20 bis 100 °C mit Phosphorpentachlorid.

**14.** Verwendung der Halogenmethylchinolinderivate der allgemeinen Formel I nach Anspruch 1 oder 2 als Zwischenprodukte zur Herstellung von Arzneimittel- und Pflanzenschutzmittelwirkstoffen.

**15.** Pharmazeutische Mittel, gekennzeichnet durch eine oder mehrere Verbindungen nach Anspruch 3 als Wirkstoffe.

## Claims

1. Halogenmethylchinoline derivatives of the general formula I

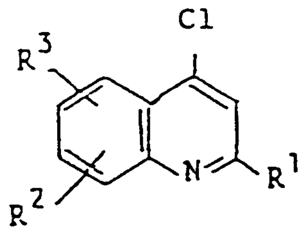

(I),

wherein
$R^1$ $-CCl_3$, $-CF_3$, $-CHCl_2$ or $-CH_2Cl$,
$R^2$ hydrogen, halogen, methoxy, methyl, $-CCl_3$, $CCF_3$ or $-CHCl_2$,
$R^3$ hydrogen , halogen, methoxy, methyl or $-CF_3$,
wherein $R^2$ and $R^3$ may not simultaneously stand for hydrogen, $R^2$ is $-CCl_3$ or $-CHCl_2$, if $R^1$ is $CF_3$, and $R^3$ is hydrogen or $-CH_3$, if $R^2$ is chlorine, and $R^2$ is hydrogen or $-CH_3$, if $R^3$ is chlorine,
as well as
2-trifluoromethyl-4,5- dichloro-8-trichloromethylchinoline.

2. 2-Trichloromethyl-4,8-dichlorochinoline,
2-trichloromethyl-6-methyl-4-chlorochinoline,
2-trichloromethyl-8-trifluoromethyl-4-chlorochinoline,
2-chloromethyl-8-methyl-4-chlorochinoline,
2-dichloromethyl-8-methyl-4-chlorochinoline,
2-trichloromethyl-8-dichloromethyl-4-chlorochinoline,
2,6-bis(trichloromethyl)-4-chlorochinoline,
2,8-bis(trichloromethyl)-4-chlorochinoline,
2-trichloromethyl-4,6-dichlorochinoline,
2-trichloromethyl-5-methyl-4-chlorochinoline,
2-trichloromethyl-8-methyl-4-chlorochinoline,
2-trichloromethyl-4,5-dichloro-8-methylchinoline,
2-trichloromethyl-4,7-dichlorochinoline,
2-trichloromethyl-6-methoxy-4-chloroxhinoline,
2-trichloromethyl-8-methoxy-4-chlorochinoline,
2-trichloromethyl-6-trifluoromethyl-4-chlorochinoline,
2-trichloromethyl-7-trifluoromethyl-4-chlorochinoline,
2-trifluoromethyl-4-chloro-5-dichloromethylchinoline,
2-trifluoromethyl-4-chloro-6-trichloromethylchinoline,
2-trifluoromethyl-4-chloro-7-trichloromethylchinoline,
2-trifluoromethyl-4-chloro-8-trichloromethylchinoline,
2-trifluoromethyl-4,5-dichloro-8-trichloromethylchinoline,
2-trifluoromethyl-4-chloro-8-trichloromethylchinoline,
2-trichloromethyl-4-chloro-7-methylchinoline,
2-trichloromethyl-4,6-dichloro-8-methylchinoline,
2-trichloromethyl-4,7-dichloro-8-methylchinoline,
2-trichloromethyl-4,8-dichloro-6-methylchinoline, and
2-dichloromethyl-4,8-dichloro-6-methylchinoline.

**3.** Halogenmethylchinoline derivatives according to claim 1 of the formula XI

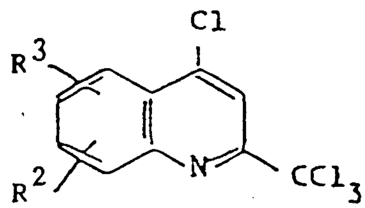

(XI),

wherein the substituents $R^2$ and $R^3$ in the below indicated combinations have the following meanings:

| $R^2$ | $R^3$ |
|---|---|
| 8-MeO | H |
| 8-Me | H |
| 8-$CCl_3$ | H |
| 5-Cl | 8-Me |
| 6-$CF_3$ | H |
| 6-MeO | H |
| 8-$CF_3$ | H |
| 6-Me | H |
| 8-$CHCl_2$ | H. |

**4.** A process for preparing the halogenmethylchinoline derivatives of the general formula I according to claim 1, characterized by the chlorination of

a) chinoline derivatives of the general formula II

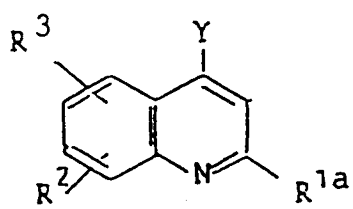

(II),

wherein

- $R^{1a}$ — $-CH_3$, $-CHCl_2$ or $-CH_2Cl$,
- $R^2$ and $R^3$ — the same as in claim 1 and
- Y — halogen or hydroxy,

b) chinoline derivatives of the general formula IV

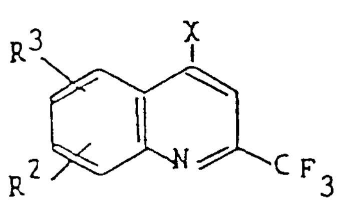

(IV),

wherein

- $R^2$ and $R^3$ — same as above and
- X — halogen, preferably chlorine or bromine,

or

c) chinoline derivatives of the general formula V

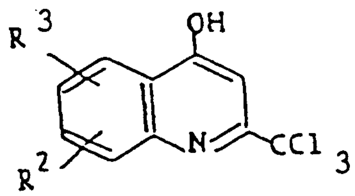

(V),

$R^2$ and $R^3$ as above,
the starting substances having at least one chlorine atom less than the corresponding final products of the formula I.

**5.** Process according to claim 4 a), characterized in that phosphorus pentachloride or phosphorus oxychloride and phosphorus pentachloride or chlorine gas or phosphorus trichloride and chlorine gas are used as chlorinating agents.

**6.** Process according to claim 5 for preparing chinoline derivatives of the general formula VI

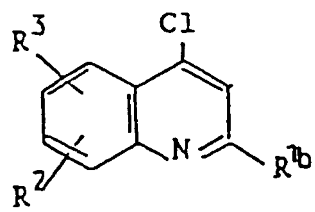

(VI),

wherein
$R^2$ and $R^3$ the same as in claim 4,
and
$R^{1b}$ $-CH_2Cl$, $-CHCl_2$ or $-CCl_3$,
characterized by
chlorination of a chinoline derivative of the general formula II according to claim 4 with phosphorus oxychloride at 70 to 140°C in the presence of halogenated hydrocarbons as solvent.

**7.** Process according to claim 5 for preparing chinoline derivatives of the general formula VII

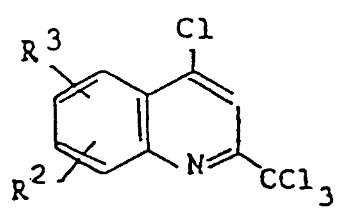

(VII),

$R^2$ and $R^3$ as in claim 4,
characterized by
the chlorination of chinoline derivatives of the general formula II according to claim 4 at 150 to 220°C and optionally under pressure in chlorinated hydrocarbons with a phosphorus trihalide and chlorine gas.

**8.** Process according to claim 6 or 7, characterized in that chlorobenzene, dichlorobenzene, trichlorobenzene or carbon tetrachloride are used as solvents.

**9.** Process according to claim 5 for preparing chinoline derivatives of the general formula VIII

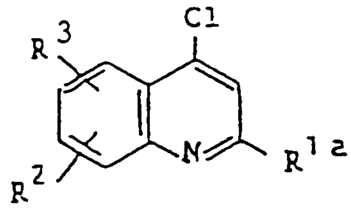

(VIII),

$R^{1a}$, $R^2$ and $R^3$ as in claim 4,

characterized by

chlorination of a chinoline derivative of the general formula IX

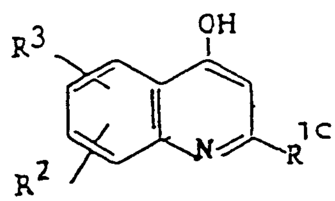

(IX),

wherein
$R^2$ and $R^3$ the same as in claim 4, and
$R^{1c}$ $-CH_3$, $-CHCl_2$, $-CH_2Cl$, $-CF_3$ or $-CCl_3$,
at 120 to 150°C with phosphorus pentachloride.

**10.** Process according to claim 9, characterized by chlorination in the presence of a halogenated hydrocarbon.

**11.** Process according to claim 4 b) for preparing chinoline derivatives of the general formula XI

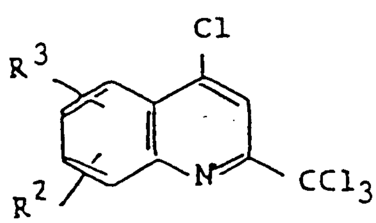

(XI),

$R^2$ and $R^3$ as in claim 1, and especially as in claim 3, characterized by

reaction of chinoline derivatives of the general formula IV, $R^2$ and $R^3$ being the same as in claim 4, with aluminum chloride.

**12.** Process according to claim 11, characterized by reaction in the presence of carbon bisulfide, nitrobenzene, or acetylchloride as solvent.

**13.** Process according to claim 4 c) for preparing chinoline derivatives of the general formula XI

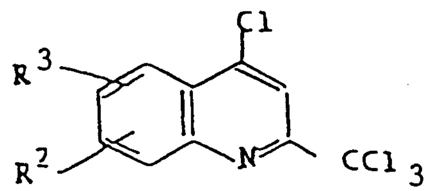

(XI),

$R^2$ and $R^3$ as in claim 1, and especially as in claim 3, characterized by chlorination of compounds of the general formula V, with $R^2$ and $R^3$ being the same as in claim 3, with phosphorus oxychloride or at 20 to 100°C with phosphorus pentachloride.

**14.** The use of the halomethyl chinoline derivatives of the general formula I according to claim 1 or 2 as intermediates for preparing pharmaceutical and plant protectant agents.

**15.** Pharmaceutical agents, characterized by one or more compounds according to claim 3 as agents.

## Revendications

**1.** Dérivés d'halogénométhylquinoléine de formule générale I

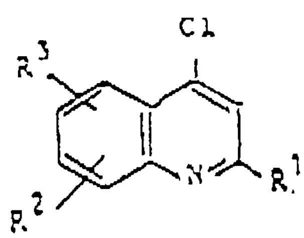

(I),

dans laquelle:

$R^1$ représente $-CCl_3$ , $-CF_3$ , $-CHCl_2$ ou $-CH_2Cl$ ,
$R^2$ représente un hydrogène, un halogène, un méthoxy, un méthyle, $-CCl_3$ , $-CF_3$ ou $-CHCl_2$ ,
$R^3$ représente un hydrogène, un halogène, un méthoxy, un méthyle ou $-CF_3$ ,

où $R^2$ et $R^3$ ne peuvent pas représenter simultanément un hydrogène, $R^2$ représente $-CCl_3$ ou $-CHCl_2$ lorsque $R^1$ représente $-CF_3$ , et $R^3$ représente un hydrogène ou $-CH_3$ lorsque $R^2$ représente un chlore, et $R^2$ représente un hydrogène ou $-CH_3$ lorsque $R^3$ est un chlore,
ainsi que
la 2-trifluorométhyl-4,5-dichloro-8-trichlorométhylquinoléine.

**2.** 2-trichlorométhyl-4,8-dichloroquinoléine,
2-trichlorométhyl-6-méthyl-4-chloroquinoléine,
2-trichlorométhyl-8-trifluorométhyl-4-chloroquinoléine,
2-chlorométhyl-8-méthyl-4-chlorochinoléine,
2-dichlorométhyl-8-méthyl-4-chloroquinoléine,
2-trichlorométhyl-8-dichlorométhyl-4-chloroquinoléine,
2,6-bis(trichlorométhyl)-4-chloroquinoléine,
2,8-bis(trichlorométhyl)-4-chloroquinoléine,
2-trichlorométhyl-4,6-dichloroquinoléine,
2-trichlorométhyl-5-méthyl-4-chloroquinoléine,
2-trichlorométhyl-8-méthyl-4-chloroquinoléine,
2-trichlorométhyl-4,5-dichloro-8-méthylquinoléine,
2-trichlorométhyl-4,7-dichloroquinoléine,
2-trichlorométhyl-6-méthyoxy-4-chloroquinoléine,
2-trichlorométhyl-8-méthoxy-4-chloroquinoléine,

2-trichlorométhyl-6-trifluorométhyl-4-chloroquinoléine,
2-trichlorométhyl-7-trifluorométhyl-4-chloroquinoléine,
2-trichlorométhyl-4-chloro-5-dichlorométhylquinoléine,
2-trichlorométhyl-4-chloro-6-trichlorométhylquinoléine,
2-trichlorométhyl-4-chloro-7-trichlorométhylquinoléine,
2-trichlorométhyl-4-chloro-8-dichlorométhylquinoléine,
2-trichlorométhyl-4,5-dichloro-8-trichlorométhylquinoléine.
2-trichlorométhyl-4-chloro-8-trichlorométhylquinoléine,
2-trichlorométhyl-4-chloro-7-méthylquinoléine,
2-trichlorométhyl-4,6-dichloro-8-méthylquinoléine,
2-trichlorométhyl-4,7-dichloro-8-méthylquinoléine,
2-trichlorométhyl-4,8-dichloro-6-méthylquinoléine
et
2-dichlorométhyl-4,8-dichloro-6-méthylquinoléine,

**3.** Dérivés d'halogénométhylquinoléine selon la revendication 1 de formule (XI)

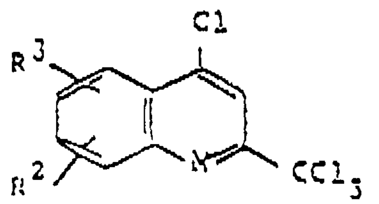

(XI),

dans laquelle les substituants $R^2$ et $R^3$ dans les combinaisons indiquées ci-dessous ont la signification suivante:

| $R^2$ | $R^3$ |
|---|---|
| 8-MeO | H |
| 8-Me | H |
| 8-$CCl_3$ | H |
| 5-Cl | 8-Me |
| 6-$CF_3$ | H |
| 6-MeO | H |
| 8-$CF_3$ | H |
| 6-Me | H |
| 8-$CHCl_2$ | H. |

**4.** Procédé de préparation des dérivés d'halogénométhylquinoléine de formule générale I selon la revendication 1, caractérisé par la chloration de
a) dérivés de quinoléine de formule générale II,

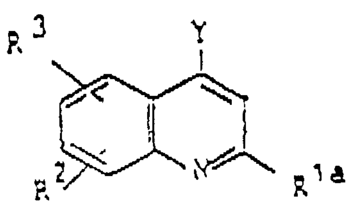

(II),

dans laquelle:
$R^{1a}$ représente $-CH_3$ , $-CHCl_2$ ou $-CH_2Cl$ ,
$R^2$ et $R^3$ ont la même signification que dans la revendication 1 et
Y représente un halogène ou un hydroxy,

b) dérivés ie quinoléine de formule générale IV

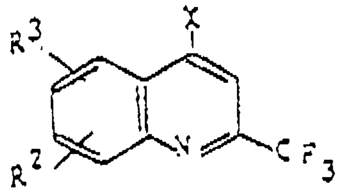

(IV),

dans laquelle:

$R^2$ et $R^3$ ont la même signification que ci-dessus et

X représente un halogène, do préférence le chlore ou le brome,

ou

c) dérivés ce quinoléine de formule générale V

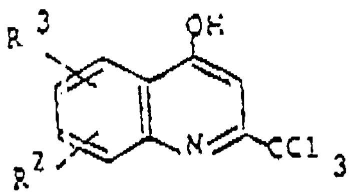

(V),

dons laquelle $R^2$ et $R^3$ ont la même signification que ci-dessus,

où las substances de départ présentent au moins un atome de chlore de moins que les produits finals de formule I correspondants.

**5.** Procédé selon la revendication 4 a), caractérisé en ce qu'on utilise comme agent de chloration le pentachlorure de phosphore ou l'oxychlorure de phosphore et le pentachlorure de phosphore ou le chlore gazeux ou le trichlorure de phosphore et le chlore gazeux.

**6.** Procédé selon la revendication 5 de préparation de dérivés de quinoléine de formule générale VI,

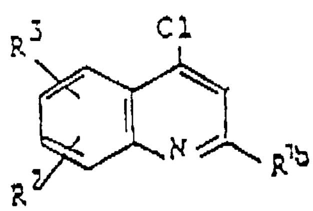

(VI),

dans laquelle

$R^2$ et $R^3$ ont la même signification que dans la revendication 4, et

$R^{1b}$ représente $-CH_2Cl$ , $-CHCl_2$ ou $-CCl_3$ ,

caractérisé par

la chloration d'un dérivé de quinoléine de formule générale II selon la revendication 4 avec de l'oxychlorure de phosphore entre 70 et 140 °C en présence d'hydrocarbures halogénés comme solvants.

**7.** Procédé selon la revendication 5 de préparation de dérivés de quinoléine de formule générale VII

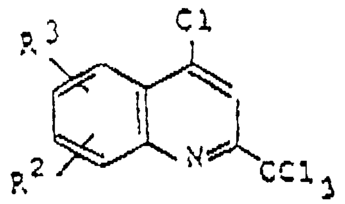

(VII),

dans laquelle $R^2$ et $R^3$ sont tels que dans la revendication 4, caractérisé par la chloration de dérivés de quinoléine de formule générale II selon la revendication 4 entre 150 et 220 °C or le cas échéant sous pression dans des hydrocarbures chlorés avec un trihalogénure de phosphore et du chlore gazeux.

**8.** Procédé selon la revendication 6 ou 7, caractérisé en ce qu'on utilise conme solvant le chlorobenzène, le dichlorobenzène, le trichlorobenzène ou le tétrachlorure de carbone.

**9.** Procédé selon la revendication 5 de préparation de dérivés de quinoléine de formule générale VIII

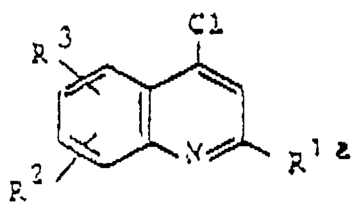

(VIII),

dans laquelle $R^{1a}$, $R^2$ et $R^3$ sont tels que dans la revendication 4, caractérisé par la chloration d'un dérivé de quinoléine de formule générale IX

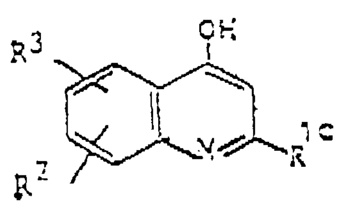

(IX),

dans laquelle:

$R^2$ et $R^3$ ont la même signification que dans la revendication 4 et

$R^{1c}$ représente $-CH_3$, $-CHCl_2$, $-CH_2Cl$, $-CF_3$ ou $-CCl_3$,

entre 120 et 150 °C avec du pentachlorure de phosphore.

**10.** Procédé selon la revendication 9, caractérisé par une chloration an présence d'un hydrocarbure halogéné.

**11.** Procédé selon la revendication 4 b) de préparation de dérivés de quinoléine de formule générale XI

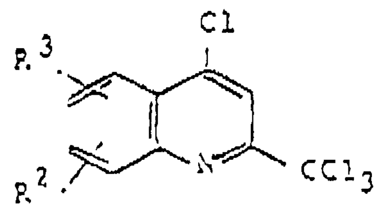

(XI),

dans laquelle $R^2$ et $R^3$ sont tels que dans la revendication 1 et en particulier dans la revendication 3, caractérisé par
la réaction de dérivés de quinoléine de formule générale IV dans laquelle $R^2$ et $R^3$ ont la même signification que dans la revendication 4, avec du chlorure d'aluminium.

**12.** Procédé selon la revendication 11, caractérisé par la réaction en présence de sulfure de carbone, de nitrobenzène ou de chlorure d'acétyle comme solvant.

**13.** Procédé selon la revendication 4 c) de préparation de dérivés de quinoléine de formule générale XI

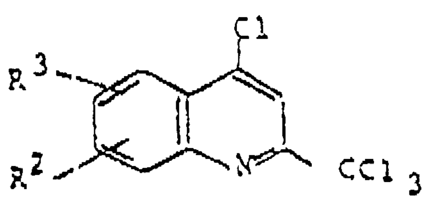

(XI),

dans laquelle $R^2$ et $R^3$ sont tels que dans la revendication 1 et en particulier dans la revendication 3, caractérisé par la chloration de composés de formule générale V, dans laquelle $R^2$ et $R^3$ ont la même signification que dans la revendication 3, avec de l'oxychlorure de phosphore ou entre 20 et 100 °C avec du peptachloruro de phosphore.

**14.** Application des dérivés d'halogénométhylquinoléine de formule générale I selon la revendication 1 ou 2 comme produits intermédiaires dans la préparation de substances actives pour médicaments et agents de protection des plantes.

**15.** Agent pharmaceutique caractérisé par un ou plusieurs composés selon la revendication 3 comme substances actives.